# EUROPÄISCHE PATENTSCHRIFT

(11) **EP 2 855 442 B1**
(45) Veröffentlichungstag und Bekanntmachung des Hinweises auf die Patenterteilung: **23.03.2016**
(21) Anmeldenummer: 13724592.4
(22) Anmeldetag: 23.05.2013
(51) Int. Cl.: C07D 295/088

(54) **VERFAHREN ZUR HERSTELLUNG EINES MONO-N-ALKYL-PIPERAZINS**
PROCESS FOR PREPARING MONO N-ALKYLPIPERAZINE
PROCÉDÉ DE PRODUCTION D'UNE MONO-N-ALKYL-PIPÉRAZINE

(30) Priorität: 01.06.2012 EP 12170569
(43) Veröffentlichungstag der Anmeldung: 08.04.2015
(73) Patentinhaber: BASF SE, 67056 Ludwigshafen (DE)
(72) Erfinder: BOU CHEDID, Roland, 68159 Mannheim (DE); MELDER, Johann-Peter, 67459 Böhl-Iggelheim (DE); ABEL, Ulrich, 67105 Schifferstadt (DE); DOSTALEK, Roman, 67271 Neuleiningen (DE); STEIN, Bernd, 64665 Alsbach-Hähnlein (DE)
(86) Internationale Anmeldenummer: PCT/EP2013/060658
(87) Internationale Veröffentlichungsnummer: WO 2013/178534

(56) Entgegenhaltungen:
- WO-A1-2009/080507
- WO-A1-2011/067199
- WO-A1-2011/157710
- WO-A1-2012/055893

## Beschreibung

Die vorliegende Erfindung betrifft ein Verfahren zur Herstellung eines Mono-N-alkyl-piperazins der Formel I 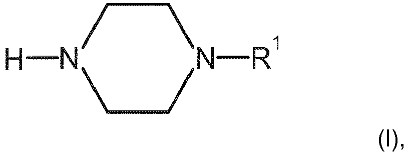 in der R¹ C₁- bis C₅-Alkyl oder 2-(2-Hydroxy-ethoxy)-ethyl bedeutet, durch Umsetzung von Diethanolamin (DEOA) der Formel II 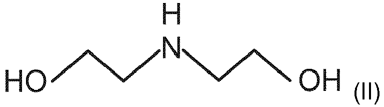 mit einem primären Amin der Formel H₂N-R¹ (III) in Gegenwart von Wasserstoff und eines geträgerten, metallhaltigen Katalysators.

Die Verfahrensprodukte finden u.a. Verwendung als Zwischenprodukte bei der Herstellung von Kraftstoffadditiven (US 3,275,554 A; DE 21 25 039 A und DE 36 11 230 A), Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren.

WO 03/051508 A1 (Huntsman Petrochemical Corp.) betrifft Verfahren zur Aminierung von Alkoholen unter Verwendung von spezifischen Cu/Ni/Zr/Sn - haltigen Katalysatoren, die in einer weiteren Ausgestaltung Cr statt Zr enthalten (siehe Seite 4, Zeilen 10-16). Die in dieser WO-Anmeldung beschriebenen Katalysatoren enthalten kein Aluminiumoxid und kein Kobalt.

WO 2008/006750 A1 (BASF AG) betrifft bestimmte Pb, Bi, Sn, Sb und/oder In - dotierte, zirkoniumdioxid-, kupfer-, nickel- und kobalthaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt.

WO 2009/080507 A1 (BASF SE) betrifft bestimmte Sn- und Co-dotierte, zirkoniumdioxid-, kupfer- und nickelhaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt.

WO 2009/080506 A1 (BASF SE) beschreibt bestimmte Pb, Bi, Sn, Mo, Sb und/oder P - dotierte, zirkoniumdioxid-, nickel- und eisenhaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt. Bevorzugt enthalten die Katalysatoren kein Cu und kein Co.

WO 2009/080508 A1 (BASF SE) lehrt bestimmte Pb, Bi, Sn und/oder Sb - dotierte, zirkoniumdioxid-, kupfer-, nickel-, kobalt- und eisenhaltige Katalysatoren und ihre Verwendung in Verfahren zur Herstellung eines Amins durch Umsetzung eines primären oder sekundären Alkohols, Aldehyds und/oder Ketons mit Wasserstoff und Ammoniak, einem primären oder sekundären Amin. Aluminiumoxid-Träger werden nicht gelehrt.

WO 2011/067199 A1 (BASF SE) betrifft bestimmte aluminiumoxid-, kupfer-, nickel-, kobalt- und zinnhaltige Katalysatoren und ihre Verwendung in Verfahren zur Herstellung eines Amins aus einem primären oder sekundären Alkohol, Aldehyd und/oder Keton. Die Herstellung von N-Methyl-piperazin aus DEOA und Monomethylamin wird auf Seite 25, Zeile 20-21, allgemein erwähnt.

WO 2011/157710 A1 (BASF SE) beschreibt die Herstellung bestimmter zyklischer tertiärer Methylamine, wobei man einen Aminoalkohol aus der Gruppe 1,4-Aminobutanol, 1,5-Aminopentanol, Aminodiglykol (ADG) bzw. Aminoethyl-ethanolamin, mit Methanol bei erhöhter Temperatur in Gegenwart eines kupferhaltigen Heterogenkatalysators in der Flüssigphase umsetzt.

WO 2012/049101 A1 (BASF SE) betrifft ein Verfahren zur Herstellung bestimmter zyklischer tertiärer Amine, indem man einen Aminoalkohol aus der Gruppe 1,4-Aminobutanol, 1,5-Aminopentanol, Aminodiglykol (ADG) bzw. Aminoethyl-ethanolamin, mit einem bestimmten primären oder sekundären Alkohol bei erhöhter Temperatur in Gegenwart eines kupferhaltigen Heterogenkatalysators in der Flüssigphase umsetzt.

CN 102 101 847 A (Zhangjiagang Tianyou New Material Techn. Co., Ltd.) beschreibt eine zweistufige Synthese für N-Methyl-N-(2-Chlorethyl)-piperazin aus Aminodiglykol (ADG) via N-Methyl-piperazin als Zwischenprodukt.

CN 102 304 101 A (Shaoxing Xingxin Chem. Co., Ltd.) betrifft die gleichzeitige Herstellung von Piperazin und N-Alkylpiperazinen durch Umsetzung von N-Hydroxyethyl-1,2-ethandiamin mit primären C₁₋₇-Alkoholen in Gegenwart von metallischen Katalysatoren.

EP 446 783 A2 (BASF AG) betrifft u.a. die Herstellung von N-arylsubstituierten Piperazinen durch Aminierung von entsprechenden N,N-Di-(2-hydroxyalkyl)-N-aryl-aminen.

EP 235 651 A1 (BASF AG) lehrt ein Verfahren zur Herstellung von N-Methyl-piperazin aus DE-OA und Methylamin in Gegenwart metallhaltiger Trägerkatalysatoren, insbesondere Cu-haltiger Katalysatoren.

Der vorliegenden Erfindung lag die Aufgabe zugrunde, die Wirtschaftlichkeit bisheriger Verfahren zur Herstellung von Mono-N-alkyl-piperazinen der Formel I zu verbessern und einem Nachteil oder mehreren Nachteilen des Stands der Technik abzuhelfen. Es sollten Bedingungen gefunden werden, die technisch in einfacher Weise herzustellen sind und die es erlauben, das Verfahren mit hohem Umsatz, hoher Ausbeute, Raum-Zeit-Ausbeuten (RZA), Selektivität bei gleichzeitig hoher mechanischer Stabilität des Katalysatorformkörpers und geringer ,Durchgehgefahr', durchzuführen.

[Raum-Zeit-Ausbeuten werden angegeben in 'Produktmenge / (Katalysatorvolumen ● Zeit)' (kg / (I_{Kat.} ● h)) und/oder,Produktmenge / (Reaktorvolumen ● Zeit)' (kg / (I_{Reaktor} ● h)].

Demgemäß wurde ein Verfahren zur Herstellung eines Mono-N-alkyl-piperazins der Formel I 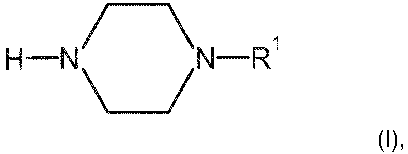 in der R¹ C₁- bis C₅-Alkyl oder 2-(2-Hydroxy-ethoxy)-ethyl bedeutet, durch Umsetzung von Diethanolamin (DEOA) der Formel II 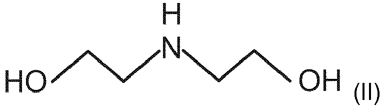 mit einem primären Amin der Formel H₂N-R¹ (III) in Gegenwart von Wasserstoff und eines geträgerten, metallhaltigen Katalysators gefunden, welches dadurch gekennzeichnet ist, dass die katalytisch aktive Masse des Katalysators vor dessen Reduktion mit Wasserstoff sauerstoffhaltige Verbindungen des Aluminiums, Kupfers, Nickels und Kobalts und im Bereich von 0,2 bis 5,0 Gew.-% sauerstoffhaltige Verbindungen des Zinns, berechnet als SnO, enthält und man die Umsetzung in der Flüssigphase bei einem Absolutdruck im Bereich von 95 bis 145 bar durchführt.

Bei dem Rest R¹ handelt es sich um 2-(2-Hydroxy-ethoxy)-ethyl oder C₁₋₅-Alkyl, bevorzugt C₁₋₃-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, besonders bevorzugt Methyl, Ethyl und 2-(2-Hydroxy-ethoxy)-ethyl.
Bei dem primären Amin III handelt es sich entsprechend besonders bevorzugt um Monomethylamin, Monoethylamin oder 1-Amino-2-(2-hydroxy-ethoxy)-ethan (Aminodiglykol, ADG).

Mit dem erfindungsgemäßen Verfahren bevorzugt herstellbar sind Amine der Formel I 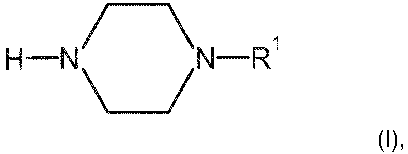 in der R¹ = Methyl, Ethyl oder 2-(2-Hydroxy-ethoxy)-ethyl bedeutet.

Insbesondere werden Katalysatoren, deren katalytisch aktive Masse vor deren Reduktion mit Wasserstoff im Bereich von
15 bis 80 Gew.-% sauerstoffhaltige Verbindungen des Aluminiums, berechnet als Al₂O₃,
1 bis 20 Gew.-% sauerstoffhaltige Verbindungen des Kupfers, berechnet als CuO,
5 bis 35 Gew.-% sauerstoffhaltige Verbindungen des Nickels, berechnet als NiO,
5 bis 35 Gew.-% sauerstoffhaltige Verbindungen des Kobalts , berechnet als CoO, und
0,2 bis 5,0 Gew.-% sauerstoffhaltige Verbindungen des Zinns, berechnet als SnO, enthält, im o. g. Aminierungsverfahren eingesetzt.

Das Verfahren kann kontinuierlich oder diskontinuierlich durchgeführt werden. Bevorzugt ist eine kontinuierliche Fahrweise.

In der Kreisgasfahrweise werden die Ausgangsstoffe (DEOA, das primäre Amin III) in einem Kreisgasstrom verdampft und gasförmig dem Reaktor zugeführt.

Die Edukte (DEOA, das primäre Amin III) können auch als wässrige Lösungen verdampft und mit dem Kreisgasstrom auf das Katalysatorbett geleitet werden.

Bevorzugte Reaktoren sind Rohrreaktoren. Beispiele für geeignete Reaktoren mit Kreisgasstrom finden sich in Ullmann's Encyclopedia of Industrial Chemistry, 5th Ed., Vol. B 4, Seiten 199-238, "Fixed-Bed Reactors".

Alternativ erfolgt die Umsetzung vorteilhaft in einem Rohrbündelreaktor oder in einer Monostranganlage.

Bei einer Monostranganlage kann der Rohrreaktor, in dem die Umsetzung erfolgt, aus einer Hintereinanderschaltung mehrerer (z. B. zweier oder dreier) einzelner Rohrreaktoren bestehen. Optional ist hier vorteilhaft eine Zwischeneinspeisung von Feed (enthaltend das DEOA und/oder primäres Amin III und/oder H₂) und/oder Kreisgas und/oder Reaktoraustrag aus einem nachgeschalteten Reaktor möglich.

Die Kreisgasmenge liegt bevorzugt im Bereich von 40 bis 1500 m³ (bei Normaldruck) / [m³ Katalysator (Schüttvolumen) • h], insbesondere im Bereich von 100 bis 1000 m³ (bei Normaldruck) / [m³ Katalysator (Schüttvolumen) • h]. (Normaldruck = 1 bar abs.)

Das Kreisgas enthält bevorzugt mindestens 10, besonders 50 bis 100, ganz besonders 80 bis 100, Vol.% Wasserstoff (H₂).

Im erfindungsgemäßen Verfahren werden die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten.
In diesem Zusammenhang wird das oxidische Trägermaterial Aluminiumoxid (Al₂O₃) als zur katalytisch aktiven Masse gehörig gewertet.

Die Katalysatoren werden dergestalt eingesetzt, dass man die katalytisch aktive, zu Pulver vermahlene Masse in das Reaktionsgefäß einbringt oder, dass man die katalytisch aktive Masse nach Mahlung, Vermischung mit Formhilfsmitteln, Formung und Temperung als Katalysatorformkörper - beispielsweise als Tabletten, Kugeln, Ringe, Extrudate (z. B. Stränge) - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und der o. g. Katalysatorträgermaterialien definiert und enthält im Wesentlichen die folgenden Bestandteile:
Aluminiumoxid (Al₂O₃), sauerstoffhaltige Verbindungen des Kupfers, Nickels und Kobalts und sauerstoffhaltige Verbindungen des Zinns.

Die Summe der o. g. Bestandteile der katalytisch aktiven Masse beträgt üblicherweise 70 bis 100 Gew.-%, bevorzugt 80 bis 100 Gew.-%, besonders bevorzugt 90 bis 100 Gew.-%, besonders > 95 Gew.-%, ganz besonders > 98 Gew.-%, insbesondere > 99 Gew.-%, z. B. besonders bevorzugt 100 Gew.-%.

Die katalytisch aktive Masse der erfindungsgemäßen und im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems, enthalten.

Beispiele für solche Elemente bzw. deren Verbindungen sind:
Übergangsmetalle, wie Mn bzw. MnO₂, W bzw. Wolframoxide, Ta bzw. Tantaloxide, Nb bzw. Nioboxide oder Nioboxalat, V bzw. Vanadiumoxide bzw. Vanadylpyrophosphat; Lanthanide, wie Ce bzw. CeO₂ oder Pr bzw. Pr₂O₃; Erdalkalimetalloxide, wie SrO; Erdalkalimetallcarbonate, wie MgCO₃, CaCO₃ und BaCO₃; Boroxid (B₂O₃).

Bevorzugt enthält die katalytisch aktive Masse der erfindungsgemäßen und im erfindungsgemäßen Verfahren eingesetzten Katalysatoren kein Rhenium, kein Ruthenium, kein Eisen und/oder kein Zink, jeweils weder in metallischer (Oxidationsstufe = 0) noch in einer ionischen (Oxidationsstufe ≠ 0), insbesondere oxidierten, Form.

Bevorzugt enthält die katalytisch aktive Masse der erfindungsgemäßen und im erfindungsgemäßen Verfahren eingesetzten Katalysatoren kein Silber und/oder Molybdän, jeweils weder in metallischer (Oxidationsstufe = 0) noch in einer ionischen (Oxidationsstufe ≠ 0), insbesondere oxidierten, Form.

In einer besonders bevorzugten Ausführungsform enthält die katalytisch aktive Masse der erfindungsgemäßen und im erfindungsgemäßen Verfahren eingesetzten Katalysatoren keine weitere katalytisch aktive Komponente, weder in elementarer (Oxidationsstufe = 0) noch in ionischer (Oxidationsstufe ≠ 0) Form.
In der besonders bevorzugten Ausführungsform ist die katalytisch aktive Masse nicht mit weiteren Metallen oder Metallverbindungen dotiert.
Bevorzugt sind jedoch aus der Metallgewinnung von Cu, Co, Ni, Sn herrührende übliche Begleit-Spurenelemente hiervon ausgenommen.

Bevorzugt enthält die katalytisch aktive Masse des Katalysators keine sauerstoffhaltigen Verbindungen des Siliziums, Zirkoniums, Titans und/oder des Chroms.

Die katalytisch aktive Masse des Katalysators enthält vor dessen Reduktion mit Wasserstoff im Bereich von 0,2 bis 5,0 Gew.-%, besonders im Bereich von 0,4 bis 4,0 Gew.-%, weiter besonders im Bereich von 0,6 bis 3,0 Gew.-%, weiter besonders bevorzugt im Bereich von 0,7 bis 2,5 Gew.-%, sauerstoffhaltige Verbindungen des Zinns, berechnet als SnO.

Die katalytisch aktive Masse des Katalysators enthält vor dessen Reduktion mit Wasserstoff bevorzugt im Bereich von 5,0 bis 35 Gew.-%, besonders im Bereich von 10 bis 30 Gew.-%, weiter besonders im Bereich von 12 bis 28 Gew.-%, ganz besonders 15 bis 25 Gew.-%, sauerstoffhaltige Verbindungen des Kobalts, berechnet als CoO.

Die katalytisch aktive Masse des Katalysators enthält vor dessen Reduktion mit Wasserstoff weiterhin bevorzugt im Bereich von
15 bis 80 Gew.-%, besonders 30 bis 70 Gew.-%, weiter besonders 35 bis 65 Gew.-%, sauerstoffhaltige Verbindungen des Aluminiums, berechnet als Al₂O₃,
1 bis 20 Gew.-%, besonders 2 bis 18 Gew.-%, weiter besonders 5 bis 15 Gew.-%, sauerstoffhaltige Verbindungen des Kupfers, berechnet als CuO, und
5 bis 35 Gew.-%, besonders 10 bis 30 Gew.-%, weiter besonders 12 bis 28 Gew.-%, ganz besonders 15 bis 25 Gew.-%, sauerstoffhaltige Verbindungen des Nickels, berechnet als NiO.

Das Molverhältnis von Nickel zu Kupfer beträgt bevorzugt größer 1, besonders bevorzugt größer 1,2, weiter besonders bevorzugt im Bereich von 1,8 bis 8,5.

Die BET-Oberfläche (ISO 9277:1995) der erfindungsgemäßen und im erfindungsgemäßen Verfahren eingesetzten Katalysatoren liegt bevorzugt im Bereich von 30 bis 250 m²/g, besonders im Bereich von 90 bis 200 m²/g, weiter besonders im Bereich von 130 bis 190 m²/g, (jeweils vor der Reduktion mit Wasserstoff). Diese Bereiche werden insbesondere durch Calcinier-Temperaturen bei der Katalysatorherstellung im Bereich von 400 bis 600 °C, besonders 420 bis 550 °C, (vgl. unten) erzielt.

Zur Herstellung der im erfindungsgemäßen Verfahren verwendeten Katalysatoren sind verschiedene Verfahren möglich. Sie sind beispielsweise durch Peptisieren pulvriger Mischungen der Hydroxide, Carbonate, Oxide und/oder anderer Salze der Komponenten mit Wasser und nachfolgendes Extrudieren und Tempern (Wärmebehandlung) der so erhaltenen Masse erhältlich.

Bevorzugt werden zur Herstellung der erfindungsgemäßen Katalysatoren Fällungsmethoden angewandt. So können sie beispielsweise durch eine gemeinsame Fällung der Nickel-, Kobalt-, Kupfer- und Sn-Komponenten aus einer diese Elemente enthaltenden, wässrigen Salzlösung mittels Basen in Gegenwart einer Aufschlämmung einer schwerlöslichen, sauerstoffhaltigen Aluminiumverbindung und anschließendes Waschen, Trocknen und Calcinieren des erhaltenen Präzipitats erhalten werden. Als schwerlösliche, sauerstoffhaltige Aluminiumverbindungen können beispielsweise Aluminiumoxid, Aluminiumoxidhydrat, Aluminiumphosphate, -borate und - silikate Verwendung finden. Die Aufschlämmungen der schwerlöslichen Aluminiumverbindungen können durch Suspendieren feinkörniger Pulver dieser Verbindungen in Wasser unter kräftigem Rühren hergestellt werden. Vorteilhaft werden diese Aufschlämmungen durch Ausfällen der schwerlöslichen Aluminiumverbindungen aus wässrigen Aluminiumsalzlösungen mittels Basen erhalten.

Bevorzugt werden die erfindungsgemäßen Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Base, - beispielsweise Natriumcarbonat, Natriumhydroxid, Kaliumcarbonat oder Kaliumhydroxid - versetzt, bis die Fällung vollständig ist. Es kann auch mit Alkalimetall-freien Basen wie Ammoniak, Ammoniumcarbonat, Ammoniumhydrogencarbonat, Ammoniumcarbamat, Ammoniumoxalat, Ammoniummalonat, Urotropin, Harnstoff, etc. gearbeitet werden. Die Art der verwendeten Salze ist im Allgemeinen nicht kritisch: Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im Allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d. h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden zu den erfindungsgemäßen Katalysatoren wie üblich weiterverarbeitet. Zunächst werden die Niederschläge gewaschen. Über die Dauer des Waschvorgangs und über die Temperatur und Menge des Waschwassers kann der Gehalt an Alkalimetall, das durch die als Fällungsmittel eventuell verwendete (Mineral)base zugeführt wurde, beeinflusst werden. Im Allgemeinen wird durch Verlängerung der Waschzeit oder Erhöhung der Temperatur des Waschwassers der Gehalt an Alkalimetall abnehmen. Nach dem Waschen wird das Fällgut im Allgemeinen bei 80 bis 200 °C, vorzugsweise bei 100 bis 150 °C, getrocknet und danach calciniert. Die Calcinierung wird im Allgemeinen bei Temperaturen zwischen 300 und 800 °C, vorzugsweise bei 400 bis 600 °C, insbesondere bei 420 bis 550 °C ausgeführt.

Die erfindungsgemäßen Katalysatoren können auch durch Tränkung von Aluminiumoxid (Al₂O₃), das beispielsweise in Form von Pulver oder Formkörpern, wie Strängen, Tabletten, Kugeln oder Ringen, vorliegt, hergestellt werden.

Das Aluminiumoxid wird beispielsweise in der amorphen, gamma-, theta- und/oder delta- Form, als Aluminiumoxohydroxid (Böhmit), bevorzugt in der amorphen Form eingesetzt.

Die Herstellung von Formkörpern kann nach den üblichen Verfahren erfolgen.

Die Tränkung erfolgt ebenfalls nach den üblichen Verfahren, wie z. B. in A. B. Stiles, Catalyst Manufacture - Laboratory and Commercial Preparations, Marcel Dekker, New York (1983) beschrieben, durch Aufbringung einer jeweils entsprechenden Metallsalzlösung in einer oder mehreren Tränkstufen, wobei als Metallsalze z. B. entsprechende Nitrate, Acetate oder Chloride verwendet werden. Die Masse wird im Anschluss an die Tränkung getrocknet und optional calziniert.

Die Tränkung kann nach der sogenannten "incipient wetness"-Methode erfolgen, bei der das Aluminiumoxid entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und optional zu calzinieren. Die mehrstufige Tränkung ist vorteilhaft besonders dann anzuwenden, wenn das Aluminiumoxid mit einer größeren Metallmenge beaufschlagt werden soll.

Zur Aufbringung der Metallkomponenten auf das Aluminiumoxid kann die Tränkung gleichzeitig mit allen Metallsalzen oder in beliebiger Reihenfolge der einzelnen Metallsalze nacheinander erfolgen.

Anschließend werden die durch Tränkung hergestellten Katalysatoren getrocknet und bevorzugt auch calciniert, z. B. bei den bereits oben angegebenen Calcinier-Temperaturbereichen.

Nach der Calcinierung wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Presse zu Formlingen, z. B. Tabletten, verpresst und tempert. Die Tempertemperaturen entsprechen dabei bevorzugt den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d. h. insbesondere als Oxide und Mischoxide.

Die z. B. wie oben beschrieben hergestellten Katalysatoren werden als solche gelagert und ggf. gehandelt. Vor ihrem Einsatz als Katalysatoren werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden.

Zur Vorreduktion werden die Katalysatoren zunächst bei bevorzugt 150 bis 200 °C über einen Zeitraum von z. B. 12 bis 20 Stunden einer Stickstoff-Wasserstoff-Atmosphäre ausgesetzt und anschließend noch bis zu ca. 24 Stunden bei bevorzugt 200 bis 400 °C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Das erfindungsgemäße Verfahren wird bevorzugt kontinuierlich durchgeführt, wobei der Katalysator bevorzugt als Festbett im Reaktor angeordnet ist. Dabei ist sowohl eine Anströmung des Katalysatorfestbetts von oben als auch von unten möglich.

Das primäre Amin III wird bevorzugt in der 0,5- bis 20-fachen molaren Menge, besonders in der 5- bis 15-fachen molaren Menge, weiter besonders in der 6- bis 13-fachen molaren Menge, insbesondere in der 7- bis 10-fachen molaren Menge, z.B. 8- bis 10-fachen molaren Menge, jeweils bezogen auf das eingesetzte DEOA, eingesetzt.
Besonders bevorzugt wird im Fall von Aminodiglykol (ADG) als primärem Amin III das primäre Amin in der 0,5- bis 2-fachen, insbesondere in der 0,6- bis 1,2-fachen, molaren Menge, jeweils bezogen auf das eingesetzte DEOA, eingesetzt.

Das primäre Amin III kann als wässrige Lösung, besonders als 30 bis 95 Gew.-%ige wässrige Lösung, z. B. auch 65 bis 90 Gew.-%ige wässrige Lösung, eingesetzt werden. Monomethylamin und Monoethylamin werden bevorzugt auch ohne weiteres Lösungsmittel (Druckgas, Reinheit besonders 95 bis 100 Gew.-%ig) eingesetzt.

Das Edukt DEOA wird bevorzugt als wässrige Lösung, besonders als 75 bis 95 Gew.-%ige wässrige Lösung, z.B. als 80 bis 85 Gew.-%ige wässrige Lösung, eingesetzt.

Bevorzugt wird eine Abgasmenge von 5 bis 800 Normkubikmeter / (Kubikmeter Katalysator • h), insbesondere 20 bis 300 Normkubikmeter / (m³ Katalysator • h), gefahren. [Normkubikmeter = auf Normalbedingungen (20 °C, 1 bar abs.) umgerechnetes Volumen].
Katalysatorvolumen-Angaben beziehen sich immer auf das Schüttvolumen.

Die Aminierung der primären Alkoholgruppen des Edukts DEOA wird in der Flüssigphase durchgeführt. Bevorzugt ist das Festbettverfahren in der Flüssigphase.

Beim kontinuierlichen Festbettverfahren in der Flüssigphase ist folgende Verfahrensausgestaltung, die sich u.a. vorteilhaft auf die Katalysatorperformance auswirkt, besonders bevorzugt. Man leitet die Edukte (DEOA, primäres Amin III) inklusive Wasserstoff zunächst bei einer Temperatur im Bereich von 80 bis 160 °C, bevorzugt 100 bis 140 °C, besonders bevorzugt 110 bis 130 °C, über den Katalysator und danach, z.B. nach 1 bis 240 Min., bevorzugt 5 bis 120 Min., besonders bevorzugt 10 bis 90 Min, weiter besonders bevorzugt 20 bis 60 Min., erhöht man die Temperatur auf 180 bis 220 °C, besonders 180 bis 215 °C, bevorzugt 185 bis 210 °C, insbesondere 190 bis 200 °C. Demnach ist also eine Prozedur zum Anfahren bei niedrigeren Temperaturen vorgeschaltet. Das aus der Anfahrprozedur resultierende Umsetzungsprodukt kann verworfen oder in die Umsetzung zurückgeführt werden.

Beim Arbeiten in der Flüssigphase leitet man die Edukte (DEOA, primäres Amin III), bevorzugt simultan, in flüssiger Phase bei Drücken von 9,5 bis 14,5 MPa (95 bis 145 bar), bevorzugt 10,0 bis 14,0 MPa, weiter bevorzugt 10,5 bis 13,5 MPa, weiter bevorzugt 11,0 bis 13,0 MPa, besonders bevorzugt 11,5 bis 12,5 MPa, und Temperaturen von im Allgemeinen 180 bis 220 °C, besonders 180 bis 215 °C, bevorzugt 185 bis 210 °C, insbesondere 190 bis 200 °C, inklusive Wasserstoff über den Katalysator, der sich üblicherweise in einem bevorzugt von außen beheizten Festbettreaktor befindet. Es ist dabei sowohl eine Rieselfahrweise als auch eine Sumpffahrweise möglich. Die Katalysatorbelastung liegt im Allgemeinen im Bereich von 0,2 bis 0,8, bevorzugt 0,3 bis 0,7, besonders bevorzugt 0,4 bis 0,6, weiter bevorzugt 0,4 bis 0,5, kg DEOA pro Liter Katalysator (Schüttvolumen) und Stunde (DEOA berechnet als 100 %ig). Gegebenenfalls kann eine Verdünnung der Edukte mit einem geeigneten Lösungsmittel, wie Wasser, Tetrahydrofuran, Dioxan, N-Methylpyrrolidon oder Ethylenglykoldimethylether, erfolgen. Es ist zweckmäßig, die Reaktanden bereits vor der Zuführung in das Reaktionsgefäß zu erwärmen, und zwar bevorzugt auf die Reaktionstemperatur.

Die Umsetzung wird bevorzugt bei einer Katalysatorbelastung im Bereich von 40 bis 1500 Normliter Wasserstoff / (I_{Kat.} • h), besonders einer Katalysatorbelastung im Bereich von 100 bis 1000 Normliter Wasserstoff / (I_{Kat.} • h), durchführt.
[Normliter = NI = auf Normalbedingungen (20 °C, 1 bar abs.) umgerechnetes Volumen].

Der Druck im Reaktionsgefäß, welcher sich aus der Summe der Partialdrücke des primären Amins III, des DEOAs und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Bei dem kontinuierlichen Arbeiten in der Flüssigphase kann das überschüssige primäre Amin III zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Das im Zuge der Umsetzung gebildete Reaktionswasser (jeweils ein Mol pro Mol umgesetzte Alkoholgruppe) wirkt sich im Allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Aus dem Reaktionsaustrag werden, nachdem dieser zweckmäßigerweise entspannt worden ist, der überschüssige Wasserstoff und das gegebenenfalls vorhandene überschüssige Aminierungsmittel entfernt und das erhaltene Reaktionsrohprodukt gereinigt, z. B. durch eine fraktionierende Rektifikation. Geeignete Aufarbeitungsverfahren sind z. B. in EP 1 312 600 A und EP 1 312 599 A (beide BASF AG) beschrieben. Das überschüssige primäre Amin und der Wasserstoff werden vorteilhaft wieder in die Reaktionszone zurückgeführt. Das gleiche gilt für die eventuell nicht vollständig umgesetztes DEOA.

Eine Aufarbeitung des Produkts der Umsetzung ist bevorzugt wie folgt ausgestaltet:
Aus dem Reaktionsprodukt der Umsetzung werden durch Destillation
   (i) zunächst ggf. unumgesetztes primäres Amin III, R¹ bevorzugt = C₁- bis C₅-Alkyl, über Kopf abgetrennt,
   (ii) Wasser über Kopf abgetrennt,
   (iii) ggf. vorhandene Nebenprodukte mit einem niedrigeren Siedepunkt als dem des Verfahrensprodukts I (Niedersieder) über Kopf abgetrennt,
   (iv) das Verfahrensprodukt Mono-N-alkyl-piperazin I über Kopf abgetrennt, wobei ggf. vorhandene Nebenprodukte mit einem höheren Siedepunkt als dem des Verfahrensprodukts I (Hochsieder) und ggf. vorhandenes unumgesetztes DEOA (II) im Sumpf verbleiben.
      Bei der Umsetzung des erfindungsgemäßen Verfahrens kann als Nebenprodukt das Alkylaminoethylethanolamin der Formel IV 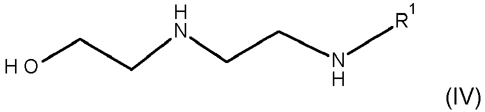 entstehen: 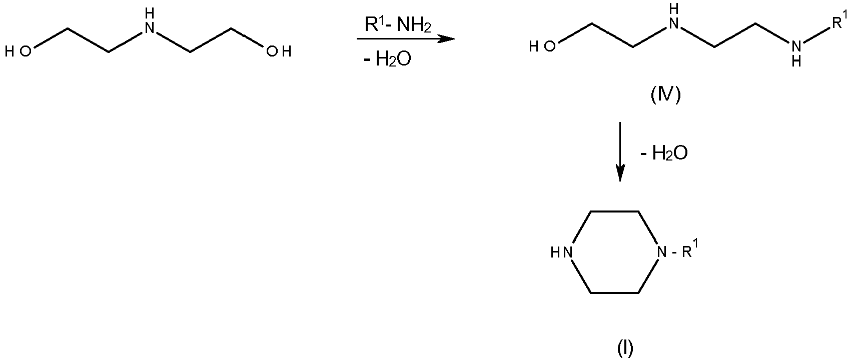 Deshalb werden im Besonderen durch Destillation
   (v) aus dem Sumpf des Schrittes iv ggf. vorhandenes unumgesetztes DEOA (II) und/oder ggf. vorhandenes Alkylaminoethylethanolamin als Nebenprodukt mit der Formel IV über Kopf abgetrennt und in die Umsetzung zurückgeführt.

In Schritt i abgetrenntes primäres Amin III mit einer Reinheit von 90 bis 99,9 Gew.-%, besonders 95 bis 99,9 Gew.-%, wird bevorzugt in die Umsetzung zurückgeführt, wobei weiter bevorzugt ein Teil des abgetrennten Amins III, besonders 1 bis 30 Gew.-% des abgetrennten Amins III, weiter besonders 5 bis 25 Gew.-% des abgetrennten Amins III, ausgeschleust wird.

Eine Aufarbeitung des Produkts der Umsetzung von Aminodiglykol (ADG), also R¹ = 2-(2-Hydroxy-ethoxy)-ethyl, mit DEOA ist bevorzugt wie folgt ausgestaltet:
Aus dem Reaktionsprodukt der Umsetzung werden durch Destillation
   (i) zunächst Wasser über Kopf abgetrennt,
   (ii) ggf. unumgesetztes ADG über Kopf abgetrennt,
   (iii) ggf. vorhandene Nebenprodukte mit einem niedrigeren Siedepunkt als dem des Verfahrensprodukts I (Niedersieder) über Kopf abgetrennt,
   (iv) das Verfahrensprodukt Mono-N-alkyl-piperazin I über Kopf abgetrennt, wobei ggf. vorhandene Nebenprodukte mit einem höheren Siedepunkt als dem des Verfahrensprodukts I (Hochsieder) und ggf. vorhandenes unumgesetztes DEOA (II) im Sumpf verbleiben.
      Im Besonderen werden durch Destillation
   (v) aus dem Sumpf des Schrittes iv ggf. vorhandenes unumgesetztes DEOA (II) und/oder ggf. vorhandenes Alkylaminoethylethanolamin als Nebenprodukt mit der Formel IV über Kopf abgetrennt und in die Umsetzung zurückgeführt.

In Schritt ii abgetrenntes ADG mit einer Reinheit von 90 bis 99,9 Gew.-%, besonders 95 bis 99,9 Gew.-%, wird bevorzugt in die Umsetzung zurückgeführt, wobei weiter bevorzugt ein Teil des abgetrennten ADG, besonders 1 bis 30 Gew.-% des abgetrennten ADG, weiter besonders 5 bis 25 Gew.-% des abgetrennten ADG, ausgeschleust wird.

Alle Druckangaben beziehen sich auf den Absolutdruck.
Alle ppm-Angaben beziehen sich auf die Masse.

### Beispiele

### 1. Herstellung des Katalysators A [= Beispiel 4 in WO 2011/067199 A (BASF SE)]

Eine wässrige Lösung aus Nickelnitrat, Kobaltnitrat, Kupfernitrat, Aluminiumnitrat, und Zinn(II)chlorid, die 3,9 Gew.-% Ni, 3,9 Gew.-% Co, 1,9 Gew.-% Cu, 5,5 Gew.-% Al₂O₃ und 0,5 Gew.-% Sn enthielt, wurde gleichzeitig in einem Rührgefäß in einem konstanten Strom mit einer 20 Gew.-%igen wässrigen Natriumcarbonatlösung bei einer Temperatur von 65-70 °C so gefällt, dass der mit einer Glaselektrode gemessene pH-Wert von 5,7 aufrechterhalten wurde. Nach der Fällung wurde für 1 Stunde Luft eingeblasen, danach wurde das pH des Lösungs mit Natriumcarbonatlösung auf den Wert 7,4 eingestellt. Die erhaltene Suspension wurde filtriert und der Filterkuchen mit vollentsalztem Wasser gewaschen bis die elektrische Leitfähigkeit des Filtrats ca. 20 mS betrug. Danach wurde der Filterkuchen bei einer Temperatur von 150 °C in einem Trockenschrank getrocknet. Das auf diese Weise erhaltene Hydroxidcarbonatgemisch wurde nun bei einer Temperatur von 500 °C über 4 Stunden calziniert. Die Katalysatormasse wurde anschließend mit 3 Gew.-% Graphit vermischt und zu Tabletten 3x3 mm verformt. Die auf dieser Weise erhaltene Tabletten werden bei einer Temperatur von 280-300 °C über mindestens 12 Stunden in Wasserstoff reduziert. Die Passivierung des reduzierten Katalysators wurde bei Raumtemperatur in verdünnter Luft (Luft in N₂ mit einem O₂-Gehalt von maximal 5 Vol.-%) durchgeführt. Der so erhaltene Katalysator hatte die Zusammensetzung wie in der folgenden Tabelle I dargestellt.

**Tabelle I**

| Katalysator *) | Ni | Co | Cu | Sn | BET **) | Träger |
|---|---|---|---|---|---|---|
| | % | % | % | % | m²/g | |
| Katalysator A | 18,6 | 17,3 | 10,6 | 1,1 | 187 | Al₂O₃ |

| | | | | | | |
|---|---|---|---|---|---|---|
| *) Katalysatorzusammensetzung in Gew.-%; Rest bis zu 100 Gew.-% ist der Träger **) ISO 9277:1995 | | | | | | |

### 2. Umsetzung von DEOA mit Monomethylamin (MMA) in einem kontinuierlich betriebenen Rohrreaktor

Ein beheizter Rohrreaktor mit 14 mm Innendurchmesser, einem zentral angebrachten Thermoelement und einem Gesamtvolumen von 1000 ml wurde im unteren Teil mit einer Schicht Glaskugeln (250 ml) befüllt, darüber mit 500 ml des Katalysators A und schließlich der restliche Teil wiederum mit Glaskugeln befüllt. Vor der Reaktion wurde der Katalysator bei max. 280 °C unter Wasserstoff (25 Nl/h) (NI = Normliter = auf Normalbedingungen (20 °C, 1 bar abs.) umgerechnetes Volumen) bei Normaldruck 24 Stunden aktiviert. Durch den Reaktor wurden von unten nach oben 300 g/h DEOA (85 %ig wässrig), 600 g/h des primären Amins und 200 Nl/h Wasserstoff dosiert. Der Reaktor wurde bei einer Temperatur von ca. 185 bis 200 °C und einem Gesamtdruck von 80-200 bar gehalten. Die Reaktionstemperatur wurde so gewählt, dass ein DE-OA Umsatz von > 90 % erreicht wurde. Das aus dem Reaktor austretende Gemisch wurde abgekühlt und auf Normaldruck entspannt. Zu verschiedenen Zeitpunkten wurden Proben vom Reaktionsgemisch genommen und mittels Gaschromatographie analysiert. Hierfür wurde eine 30 m lange GC Säule "RTX-5 Amine" verwendet, mit einem Temperaturprogramm: 70 °C/5 Min., aufheizen auf 280 °C mit einer Geschwindigkeit von 5 °C/Min., bei 280 °C/10 Minuten.

Bei 200 bar, 400 NI / (l•h) Wasserstoff, einem Molverhältnis (MV) MMA:DEOA von 10, einer DEOA-Belastung von 0,5 kg / (l•h) (ber. 100 %ig, als 85 %ige Lösung) wurde nach Erreichen einer Temperatur von 195 °C in 25 Minuten eine Durchgehreaktion beobachtet: Die Temperatur stieg spontan auf 253 °C und der Druck auf 268 bar. Zersetzungen finden unter diese Bedingungen statt und es entstehen Gase (wie Methan), die zur Druckerhöhung führen. Diese Bedingungen können sicherheitstechnisch nicht nachhaltig ausgeübt werden. In anderen Versuchen bei reduziertem Druck von z.B. ca. 120 bar wurden solche Durchgehreaktionen nicht beobachtet.

Bei 80 bar, 400 Nl / (l•h) Wasserstoff, einem Molverhältnis (MV) MMA:DEOA von 10, einer DE-OA-Belastung von 0,5 kg / (l • h) (ber. 100 %ig, als 85 %ige Lösung) wurde bei einer Temperatur von 195 °C und einem Druck von nur 80 bar ein Selektivitätsabfall (bez. N-Methyl-PIP) beobachtet.

Die Ergebnisse der Versuche bei 80-120 bar sind der folgenden Tabelle II zu entnehmen. Der untere Teil der Tabelle zeigt die Zusammensetzungen der Reaktionsausträge per GC-Analyse.

**Tabelle II**

| | Kat. | Druck | H₂ | MV MMA:DEOA | Temp. | Zulauf DEOA | Belastung ber. 100 %ig DEOA | Umsatz DEOA | Sel. NMePIP bez. auf DEOA mol% |
|---|---|---|---|---|---|---|---|---|---|
| | | bar | Nl / (l•h) | mol/mol | °C | *) | kg / (l • h) | mol% | |
| B1 | A | 120 | 150 | 10 | 195 | 85 %ig | 0,5 | 88 | 72 |
| B2 | A | 120 | 400 | 10 | 195 | 85 %ig | 0,5 | 97 | 73 |
| B3 | A | 120 | 400 | 12 | 195 | 85 %ig | 0,5 | 90 | 71 |
| B4 | A | 80 | 400 | 10 | 195 | 85 %ig | 0,5 | 91 | 62 |
| | | | | | | | | | |

| | | Leichtsieder | Piperazin | NMePIP | Dimethylpiperazin | DEOA | Verbindung IV | Hochsieder | |
|---|---|---|---|---|---|---|---|---|---|
| | B1 | 6,9 % | 1,6 % | 63,4 % | 0,8 % | 12,4 % | 9,5 % | 5,5 % | |
| | B2 | 7,5 % | 1,6 % | 70,8 % | 0,9 % | 2,5 % | 6,6 % | 10,1 % | |
| | B3 | 8 % | 1,3 % | 63,9 % | 0,7 % | 10,6 % | 8,0 % | 7,5 % | |
| | B4 | 9,3 % | 1,7 % | 56,4 % | 1,6 % | 9,2 % | 12,6 % | 9,2 % | |

| | | | | | | | | | |
|---|---|---|---|---|---|---|---|---|---|
| Kat.: Katalysator Temp.: Temperatur im Reaktor Bel.: Katalysatorbelastung [kg DEOA / (Liter_{Kat.} • h)] MV: Molverhältnis im Feed Sel.: Selektivität NMePIP: Monomethylpiperazin (N-Methyl-PIP) *): wässrige Lösung, in Gew.-% | | | | | | | | | |

### 3. Umsetzung von DEOA mit Aminodiglykol (ADG, 1-Amino-2-(2-hydroxy-ethoxy)-ethan) in einem Batchreaktor

Ein Batchreaktor mit Rührer, einem Thermoelement und einem Gesamtvolumen von 300 ml wurde gefüllt mit 7,5 g aktiviertem Katalysator. Dazu wurde der Katalysator bei max. 200 °C unter Wasserstoff (25 Nl/h) [Nl = Normliter = auf Normalbedingungen (20 °C, 1 bar abs.) umgerechnetes Volumen] bei Normaldruck 24 Stunden aktiviert. Das Eduktgemisch von DEOA und ADG wurde vorgelegt und der Reaktor wurde aufgeheizt auf 180 °C. Das gesamten Reaktionsgemisch wurde dann mit 200 bar Wasserstoff beaufschlagt. Zu verschiedenen Zeitpunkten wurden Proben vom Reaktionsgemisch genommen und mittels Gaschromatographie analysiert. Hierfür wurde eine 30 m lange GC Säule "RTX-5 Amine" verwendet, mit einem Temperaturprogramm: 70 °C/5 Min., aufheizen auf 280 °C mit einer Geschwindigkeit von 5 °C/Min., bei 280 °C/10 Minuten.
Die Ergebnisse der Versuche sind der folgenden Tabelle III zu entnehmen.

**Tabelle III**

| Kat. | Druck bar | Temp. | Zeit (h) | DEOA (g) | MV ADG:DEOA mol/mol | Umsatz DEOA | Umsatz ADG | Sel.. HE OEtPIP bez. DEOA (mol%) | Sel. HEOEtPIP bez. ADG (mol%) |
|---|---|---|---|---|---|---|---|---|---|
| A | 200 | 180 | 5 | 77 | 1 | 29 | 36 | 6 | 7 |
| A | 200 | 180 | 10 | 77 | 1 | 56 | 62 | 13 | 18 |
| A | 200 | 180 | 15 | 77 | 1 | 81 | 86 | 24 | 36 |

| | | | | | | | | | |
|---|---|---|---|---|---|---|---|---|---|
| Kat.: Katalysator MV: Molverhältnis im Feed Sel.: Selektivität (mol%) Umsatz: mol% HEOEtPIP: 2-(2-Hydroxy-ethoxy)-ethyl-piperazin | | | | | | | | | |

### 4. Aufarbeitung

Die Aufarbeitung kann bevorzugt durch die folgenden fünf Schritte erfolgen (hier am Beispiel einer Umsetzung von DEOA mit Monomethylamin oder Monoethylamin):
1) Abtrennung von unumgesetztem primären Amin (Monomethylamin bzw. Monoethylamin) und Rückführung in dem Reaktor
   Ggf. Ausschleusung eines Teils des Monomethylamins bzw. Monoethylamins vom Kopf der Kolonne.
2) Abtrennung von Wasser
3) Abtrennung von leicht siedenden Nebenkomponenten
4) Reindestillation des N-Alkyl-piperazins I über Kopf, dabei Abtrennung von hoch siedenden Nebenkomponenten über Sumpf.
5) Ggf. Rückführung eines Teils der hochsiedenden Nebenkomponenten, insbesondere Diethanolamin, N-(N'-Methyl-2-aminoethyl)-ethanolamin, N-Methyl-N-(2-Aminoethyl)-ethanolamin (bzw. N-(N'-Ethyl-2-aminoethyl)-ethanolamin, N-Ethyl-N-(2-Aminoethyl)-ethanolamin), in die Umsetzung.

## Patentansprüche

1. Verfahren zur Herstellung eines Mono-N-alkyl-piperazins der Formel I 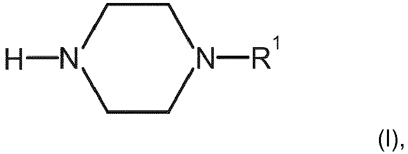 in der R¹ C₁- bis C₅-Alkyl oder 2-(2-Hydroxy-ethoxy)-ethyl bedeutet, durch Umsetzung von Diethanolamin (DEOA) der Formel II 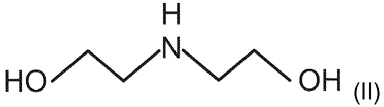 mit einem primären Amin der Formel H₂N-R¹ (III) in Gegenwart von Wasserstoff und eines geträgerten, metallhaltigen Katalysators, **dadurch gekennzeichnet, dass** die katalytisch aktive Masse des Katalysators vor dessen Reduktion mit Wasserstoff sauerstoffhaltige Verbindungen des Aluminiums, Kupfers, Nickels und Kobalts und im Bereich von 0,2 bis 5,0 Gew.-% sauerstoffhaltige Verbindungen des Zinns, berechnet als SnO, enthält und man die Umsetzung in der Flüssigphase bei einem Absolutdruck im Bereich von 95 bis 145 bar durchführt.

2. Verfahren nach Anspruch 1, **dadurch gekennzeichnet, dass** die katalytisch aktive Masse des Katalysators vor dessen Reduktion mit Wasserstoff im Bereich von 0,4 bis 4,0 Gew.-% sauerstoffhaltige Verbindungen des Zinns, berechnet als SnO, enthält.

3. Verfahren nach Anspruch 1, **dadurch gekennzeichnet, dass** die katalytisch aktive Masse des Katalysators vor dessen Reduktion mit Wasserstoff im Bereich von 0,6 bis 3,0 Gew.-% sauerstoffhaltige Verbindungen des Zinns, berechnet als SnO, enthält.

4. Verfahren nach einem der vorhergehenden Ansprüche, **dadurch gekennzeichnet, dass** die katalytisch aktive Masse des Katalysators vor dessen Reduktion mit Wasserstoff im Bereich von 5,0 bis 35 Gew.-% sauerstoffhaltige Verbindungen des Kobalts, berechnet als CoO, enthält.

5. Verfahren nach einem der vorhergehenden Ansprüche, **dadurch gekennzeichnet, dass** die katalytisch aktive Masse des Katalysators vor dessen Reduktion mit Wasserstoff im Bereich von 10 bis 30 Gew.-% sauerstoffhaltige Verbindungen des Kobalts, berechnet als CoO, enthält.

6. Verfahren nach einem der vorhergehenden Ansprüche, **dadurch gekennzeichnet, dass** die katalytisch aktive Masse des Katalysators vor dessen Reduktion mit Wasserstoff im Bereich von
15 bis 80 Gew.-% sauerstoffhaltige Verbindungen des Aluminiums, berechnet als Al₂O₃,
1,0 bis 20 Gew.-% sauerstoffhaltige Verbindungen des Kupfers, berechnet als CuO, und
5,0 bis 35 Gew.-% sauerstoffhaltige Verbindungen des Nickels, berechnet als NiO, enthält.

7. Verfahren nach einem der Ansprüche 1 bis 5, **dadurch gekennzeichnet, dass** die katalytisch aktive Masse des Katalysators vor dessen Reduktion mit Wasserstoff im Bereich von 30 bis 70 Gew.-% sauerstoffhaltige Verbindungen des Aluminiums, berechnet als Al₂O₃, 2,0 bis 18 Gew.-% sauerstoffhaltige Verbindungen des Kupfers, berechnet als CuO, und 10 bis 30 Gew.-% sauerstoffhaltige Verbindungen des Nickels, berechnet als NiO, enthält.

8. Verfahren nach einem der vorhergehenden Ansprüche, **dadurch gekennzeichnet, dass** im Katalysator das Molverhältnis von Nickel zu Kupfer größer 1 ist.

9. Verfahren nach einem der vorhergehenden Ansprüche, **dadurch gekennzeichnet, dass** die katalytisch aktive Masse des Katalysators kein Rhenium und/oder Ruthenium enthält.

10. Verfahren nach einem der vorhergehenden Ansprüche, **dadurch gekennzeichnet, dass** die katalytisch aktive Masse des Katalysators kein Eisen und/oder Zink enthält.

11. Verfahren nach einem der vorhergehenden Ansprüche, **dadurch gekennzeichnet, dass** die katalytisch aktive Masse des Katalysators keine sauerstoffhaltigen Verbindungen des Siliziums und/oder des Zirkoniums und/oder des Titans enthält.

12. Verfahren nach einem der vorhergehenden Ansprüche, **dadurch gekennzeichnet, dass** die BET-Oberfläche des Katalysators (ISO 9277:1995) im Bereich von 30 bis 250 m²/g beträgt.

13. Verfahren nach einem der vorhergehenden Ansprüche, **dadurch gekennzeichnet, dass** man die Umsetzung bei einer Temperatur im Bereich von 180 bis 220 °C durchführt.

14. Verfahren nach einem der vorhergehenden Ansprüche, **dadurch gekennzeichnet, dass** man die Umsetzung bei einem Absolutdruck im Bereich von 100 bis 140 bar durchführt.

15. Verfahren nach einem der vorhergehenden Ansprüche, **dadurch gekennzeichnet, dass** das primäre Amin III in der 5- bis 15-fachen molaren Menge bezogen auf das eingesetzte DEOA eingesetzt wird.

16. Verfahren nach einem der vorhergehenden Ansprüche 1 bis 14, **dadurch gekennzeichnet, dass** Aminodiglykol (ADG) in der 0,2- bis 2-fachen molaren Menge bezogen auf das eingesetzte DEOA eingesetzt wird.

17. Verfahren nach einem der vorhergehenden Ansprüche, **dadurch gekennzeichnet, dass** der Katalysator im Reaktor als Festbett angeordnet ist.

18. Verfahren nach einem der vorhergehenden Ansprüche, **dadurch gekennzeichnet, dass** es kontinuierlich durchgeführt wird.

19. Verfahren nach den beiden vorhergehenden Ansprüchen, **dadurch gekennzeichnet, dass** man die Umsetzung zunächst bei einer Temperatur im Bereich von 80 bis 160 °C und danach bei einer Temperatur im Bereich von 180 bis 220 °C durchführt.

20. Verfahren nach einem der drei vorhergehenden Ansprüche, **dadurch gekennzeichnet, dass** die Umsetzung in einem Rohrreaktor erfolgt.

21. Verfahren nach einem der vier vorhergehenden Ansprüche, **dadurch gekennzeichnet, dass** die Umsetzung bei einer Kreisgasfahrweise erfolgt.

22. Verfahren nach einem der vorhergehenden Ansprüche, **dadurch gekennzeichnet, dass** man das DEOA als wässrige Lösung einsetzt.

23. Verfahren nach einem der vorhergehenden Ansprüche, **dadurch gekennzeichnet, dass** man das primäre Amin III als wässrige Lösung einsetzt.

24. Verfahren nach einem der vorhergehenden Ansprüche, **dadurch gekennzeichnet, dass** man die Umsetzung bei einer Katalysatorbelastung im Bereich von 0,3 bis 0,7 kg DEOA / (I_{Kat.} ● h) durchführt.

25. Verfahren nach einem der vorhergehenden Ansprüche, **dadurch gekennzeichnet, dass** man die Umsetzung bei einer Katalysatorbelastung im Bereich von 100 bis 1000 Normliter Wasserstoff / (I_{Kat.} ● h) durchführt.

26. Verfahren nach einem der vorhergehenden Ansprüche zur Herstellung eines Mono-N-alkyl-piperazins der Formel I, in der R¹ Methyl, Ethyl oder 2-(2-Hydroxy-ethoxy)-ethyl bedeutet, durch Umsetzung von Diethanolamin (DEOA) der Formel II mit einem primären Amin der Formel H₂N-R¹ (III).

27. Verfahren nach einem der vorhergehenden Ansprüche, **dadurch gekennzeichnet, dass** aus dem Reaktionsprodukt der Umsetzung durch Destillation
(i) zunächst ggf. unumgesetztes primäres Amin III über Kopf abgetrennt wird,
(ii) Wasser über Kopf abgetrennt wird,
(iii) ggf. vorhandene Nebenprodukte mit einem niedrigeren Siedepunkt als dem des Verfahrensprodukts I über Kopf abgetrennt werden,
(iv) das Verfahrensprodukt Mono-N-alkyl-piperazin I über Kopf abgetrennt wird, wobei ggf.
vorhandene Nebenprodukte mit einem höheren Siedepunkt als dem des Verfahrensprodukts I und ggf. vorhandenes unumgesetztes DEOA (II) im Sumpf verbleiben.

28. Verfahren nach dem vorhergehenden Anspruch, **dadurch gekennzeichnet, dass** durch Destillation
(v) aus dem Sumpf des Schrittes iv ggf. vorhandenes unumgesetztes DEOA (II) und/oder ggf. vorhandenes Alkylaminoethylethanolamin als Nebenprodukt mit der Formel IV 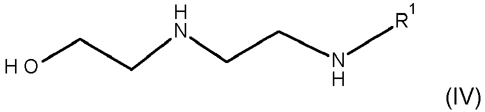 über Kopf abgetrennt und in die Umsetzung zurückgeführt werden.

29. Verfahren nach einem der beiden vorhergehenden Ansprüche, **dadurch gekennzeichnet, dass** in Schritt i abgetrenntes primäres Amin III mit einer Reinheit von 90 bis 99,9 Gew.-% in die Umsetzung zurückgeführt wird, wobei bevorzugt ein Teil des Amins III ausgeschleust wird.

30. Verfahren nach einem der Ansprüche 1 bis 26 zur Herstellung von Mono-N-alkyl-piperazin der Formel I mit R¹ = 2-(2-Hydroxy-ethoxy)-ethyl, **dadurch gekennzeichnet, dass** aus dem Reaktionsprodukt der Umsetzung durch Destillation
(i) zunächst Wasser über Kopf abgetrennt wird,
(ii) ggf. unumgesetztes primäres Amin III (= ADG) über Kopf abgetrennt wird,
(iii) ggf. vorhandene Nebenprodukte mit einem niedrigeren Siedepunkt als dem des Verfahrensprodukts I über Kopf abgetrennt werden,
(iv) das Verfahrensprodukt Mono-N-alkyl-piperazin I über Kopf abgetrennt wird, wobei ggf. vorhandene Nebenprodukte mit einem höheren Siedepunkt als dem des Verfahrensprodukts I und ggf. vorhandenes unumgesetztes DEOA (II) im Sumpf verbleiben.

31. Verfahren nach dem vorhergehenden Anspruch, **dadurch gekennzeichnet, dass** durch Destillation
(v) aus dem Sumpf des Schrittes iv ggf. vorhandenes unumgesetztes DEOA (II) und/oder ggf. vorhandenes Alkylaminoethylethanolamin als Nebenprodukt mit der Formel IV 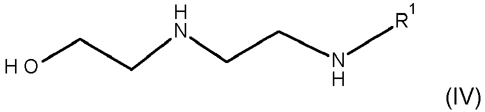 über Kopf abgetrennt und in die Umsetzung zurückgeführt werden.

32. Verfahren nach einem der beiden vorhergehenden Ansprüche, **dadurch gekennzeichnet, dass** in Schritt ii abgetrenntes ADG mit einer Reinheit von 90 bis 99,9 Gew.-% in die Umsetzung zurückgeführt wird, wobei bevorzugt ein Teil des ADGs ausgeschleust wird.

## Claims

1. A process for the preparation of a mono-N-alkylpiperazine of the formula I 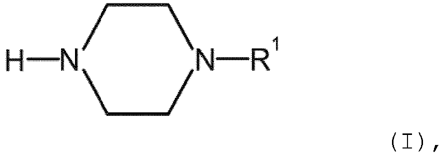 in which R¹ is C₁- to C₅-alkyl or 2-(2-hydroxyethoxy)ethyl,
by reacting diethanolamine (DEOA) of the formula II 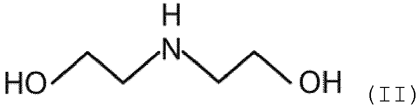 with a primary amine of the formula H₂N-R¹ (III) in the presence of hydrogen and a supported, metal-containing catalyst, wherein the catalytically active mass of the catalyst, prior to its reduction with hydrogen, comprises oxygen-containing compounds of aluminum, copper, nickel and cobalt and, in the range from 0.2 to 5.0% by weight, oxygen-containing compounds of tin, calculated as SnO, and the reaction is carried out in the liquid phase at an absolute pressure in the range from 95 to 145 bar.

2. The process according to claim 1, wherein the catalytically active mass of the catalyst, prior to its reduction with hydrogen, comprises in the range from 0.4 to 4.0% by weight of oxygen-containing compounds of tin, calculated as SnO.

3. The process according to claim 1, wherein the catalytically active mass of the catalyst, prior to its reduction with hydrogen, comprises in the range from 0.6 to 3.0% by weight of oxygen-containing compounds of tin, calculated as SnO.

4. The process according to any one of the preceding claims, wherein the catalytically active mass of the catalyst, prior to its reduction with hydrogen, comprises in the range from 5.0 to 35% by weight of oxygen-containing compounds of cobalt, calculated as CoO.

5. The process according to any one of the preceding claims, wherein the catalytically active mass of the catalyst, prior to its reduction with hydrogen, comprises in the range from 10 to 30% by weight of oxygen-containing compounds of cobalt, calculated as CoO.

6. The process according to any one of the preceding claims, wherein the catalytically active mass of the catalyst, prior to its reduction with hydrogen, comprises in the range from
15 to 80% by weight of oxygen-containing compounds of aluminum, calculated as Al₂O₃,
1.0 to 20% by weight of oxygen-containing compounds of copper, calculated as CuO, and
5.0 to 35% by weight of oxygen-containing compounds of nickel, calculated as NiO.

7. The process according to any one of claims 1 to 5, wherein the catalytically active mass of the catalyst, prior to its reduction with hydrogen, comprises in the range from
30 to 70% by weight of oxygen-containing compounds of aluminum, calculated as Al₂O₃,
2.0 to 18% by weight of oxygen-containing compounds of copper, calculated as CuO, and
10 to 30% by weight of oxygen-containing compounds of nickel, calculated as NiO.

8. The process according to any one of the preceding claims, wherein, in the catalyst, the molar ratio of nickel to copper is greater than 1.

9. The process according to any one of the preceding claims, wherein the catalytically active mass of the catalyst comprises no rhenium and/or ruthenium.

10. The process according to any one of the preceding claims, wherein the catalytically active mass of the catalyst comprises no iron and/or zinc.

11. The process according to any one of the preceding claims, wherein the catalytically active mass of the catalyst comprises no oxygen-containing compounds of silicon and/or of zirconium and/or of titanium.

12. The process according to any one of the preceding claims, wherein the BET surface area of the catalyst (ISO 9277:1995) is in the range from 30 to 250 m²/g.

13. The process according to any one of the preceding claims, wherein the reaction is carried out at a temperature in the range from 180 to 220°C.

14. The process according to any one of the preceding claims, wherein the reaction is carried out at an absolute pressure in the range from 100 to 140 bar.

15. The process according to any one of the preceding claims, wherein the primary amine III is used in a 5- to 15-fold molar amount, based on the DEOA used.

16. The process according to any one of the preceding claims 1 to 14, wherein aminodiglycol (ADG) is used in a 0.2- to 2-fold molar amount, based on the DEOA used.

17. The process according to any one of the preceding claims, wherein the catalyst is arranged as a fixed bed in the reactor.

18. The process according to any one of the preceding claims, which is carried out continuously.

19. The process according to the two preceding claims, wherein the reaction is carried out firstly at a temperature in the range from 80 to 160°C and then at a temperature in the range from 180 to 220°C.

20. The process according to any one of the three preceding claims, wherein the reaction takes place in a tubular reactor.

21. The process according to any one of the four preceding claims, wherein the reaction takes place in a circulating-gas mode.

22. The process according to any one of the preceding claims, wherein the DEOA is used as aqueous solution.

23. The process according to any one of the preceding claims, wherein the primary amine III is used as aqueous solution.

24. The process according to any one of the preceding claims, wherein the reaction is carried out at a catalyst hourly space velocity in the range from 0.3 to 0.7 kg of DEOA / (l_{cat.} ● h).

25. The process according to any one of the preceding claims, wherein the reaction is carried out at a catalyst hourly space velocity in the range from 100 to 1000 liters (stp) of hydrogen/ (l_{cat.} ● h).

26. The process according to any one of the preceding claims for the preparation of a mono-N-alkylpiperazine of the formula I in which R¹ is methyl, ethyl or 2-(2-hydroxyethoxy)ethyl, by reacting diethanolamine (DEOA) of the formula II with a primary amine of the formula H₂N-R¹ (III).

27. The process according to any one of the preceding claims, wherein, from the reaction product of the reaction, by distillation,
(i) firstly optionally unreacted primary amine III is separated off overhead,
(ii) water is separated off overhead,
(iii) optionally present by-products with a lower boiling point than that of the process product I are separated off overhead,
(iv) the process product mono-N-alkylpiperazine I is separated off overhead, with optionally present by-products with a higher boiling point than that of the process product I and optionally present unreacted DEOA (II) remaining in the bottom.

28. The process according to the preceding claim, wherein, by distillation,
(v) from the bottom of step iv, optionally present unreacted DEOA (II) and/or optionally present alkylaminoethylethanolamine as by-product with the formula IV 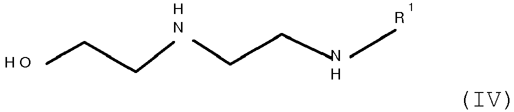 are separated off overhead and returned to the reaction.

29. The process according to either of the two preceding claims, wherein primary amine III separated off in step i and having a purity of from 90 to 99.9% by weight is returned to the reaction, with preferably some of the amine III being removed.

30. The process according to any one of claims 1 to 26 for preparing mono-N-alkylpiperazine of the formula I where R¹ = 2-(2-hydroxyethoxy)ethyl, wherein, from the reaction product of the reaction, by distillation,
(i) firstly water is separated off overhead,
(ii) optionally unreacted primary amine III (= ADG) is separated off overhead,
(iii) optionally present by-products with a lower boiling point than that of the process product I are separated off overhead,
(iv) the process product mono-N-alkylpiperazine I is separated off overhead, with optionally present by-products with a higher boiling point than that of the process product I and optionally present unreacted DEOA (II) remaining in the bottom.

31. The process according to the preceding claim, wherein, by distillation,
(v) from the bottom of step iv, optionally present unreacted DEOA (II) and/or optionally present alkylaminoethylethanolamine as by-product with the formula IV 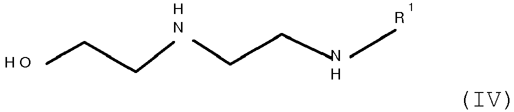 are separated off overhead and returned to the reaction.

32. The process according to either of the two preceding claims, wherein ADG separated off in step ii and having a purity of from 90 to 99.9% by weight is returned to the reaction, with preferably some of the ADG being removed.

## Revendications

1. Procédé de fabrication d'une mono-N-alkyl-pipérazine de formule I 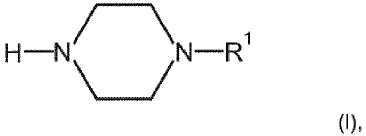 dans laquelle R¹ signifie alkyle en C₁ à C₅ ou 2-(2-hydroxy-éthoxy)-éthyle,
par mise en réaction de diéthanolamine (DEOA) de formule II 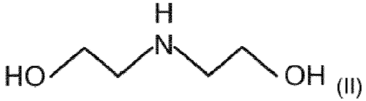 avec une amine primaire de formule H₂N-R¹ (III) en présence d'hydrogène et d'un catalyseur supporté contenant un métal, **caractérisé en ce que** la masse catalytiquement active du catalyseur contient avant sa réduction avec de l'hydrogène des composés oxygénés d'aluminium, de cuivre, de nickel et de cobalt, et dans la plage allant de 0,2 à 5,0 % en poids de composés oxygénés d'étain, calculés en tant que SnO, et la réaction est réalisée en phase liquide à une pression absolue dans la plage allant de 95 à 145 bar.

2. Procédé selon la revendication 1, **caractérisé en ce que** la masse catalytiquement active du catalyseur contient avant sa réduction avec de l'hydrogène dans la plage allant de 0,4 à 4,0 % en poids de composés oxygénés d'étain, calculés en tant que SnO.

3. Procédé selon la revendication 1, **caractérisé en ce que** la masse catalytiquement active du catalyseur contient avant sa réduction avec de l'hydrogène dans la plage allant de 0,6 à 3,0 % en poids de composés oxygénés d'étain, calculés en tant que SnO.

4. Procédé selon l'une quelconque des revendications précédentes, **caractérisé en ce que** la masse catalytiquement active du catalyseur contient avant sa réduction avec de l'hydrogène dans la plage allant de 5,0 à 35 % en poids de composés oxygénés de cobalt, calculés en tant que CoO.

5. Procédé selon l'une quelconque des revendications précédentes, **caractérisé en ce que** la masse catalytiquement active du catalyseur contient avant sa réduction avec de l'hydrogène dans la plage allant de 10 à 30 % en poids de composés oxygénés de cobalt, calculés en tant que CoO.

6. Procédé selon l'une quelconque des revendications précédentes, **caractérisé en ce que** la masse catalytiquement active du catalyseur contient avant sa réduction avec de l'hydrogène dans la plage allant de
15 à 80 % en poids de composés oxygénés d'aluminium, calculés en tant qu'Al₂O₃,
1,0 à 20 % en poids de composés oxygénés de cuivre, calculés en tant que CuO, et
5,0 à 35 % en poids de composés oxygénés de nickel, calculés en tant que NiO.

7. Procédé selon l'une quelconque des revendications 1 à 5, **caractérisé en ce que** la masse catalytiquement active du catalyseur contient avant sa réduction avec de l'hydrogène dans la plage allant de 30 à 70 % en poids de composés oxygénés d'aluminium, calculés en tant qu'Al₂O₃, 2,0 à 18 % en poids de composés oxygénés de cuivre, calculés en tant que CuO, et 10 à 30 % en poids de composés oxygénés de nickel, calculés en tant que NiO.

8. Procédé selon l'une quelconque des revendications précédentes, **caractérisé en ce que**, dans le catalyseur, le rapport molaire entre le nickel et le cuivre est supérieur à 1.

9. Procédé selon l'une quelconque des revendications précédentes, **caractérisé en ce que** la masse catalytiquement active du catalyseur ne contient pas de rhénium et/ou de ruthénium.

10. Procédé selon l'une quelconque des revendications précédentes, **caractérisé en ce que** la masse catalytiquement active du catalyseur ne contient pas de fer et/ou de zinc.

11. Procédé selon l'une quelconque des revendications précédentes, **caractérisé en ce que** la masse catalytiquement active du catalyseur ne contient pas de composés oxygénés de silicium et/ou de zirconium et/ou de titane.

12. Procédé selon l'une quelconque des revendications précédentes, **caractérisé en ce que** la surface BET du catalyseur (ISO 9277:1995) est dans la plage allant de 30 à 250 m²/g.

13. Procédé selon l'une quelconque des revendications précédentes, **caractérisé en ce que** la réaction est réalisée à une température dans la plage allant de 180 à 220 °C.

14. Procédé selon l'une quelconque des revendications précédentes, **caractérisé en ce que** la réaction est réalisée à une pression absolue dans la plage allant de 100 à 140 bar.

15. Procédé selon l'une quelconque des revendications précédentes, **caractérisé en ce que** l'amine primaire III est utilisé en une quantité molaire de 5 à 15 fois par rapport à la DEOA utilisée.

16. Procédé selon l'une quelconque des revendications 1 à 14, **caractérisé en ce que** l'aminodiglycol (ADG) est utilisé en une quantité molaire de 0,2 à 2 fois par rapport à la DEOA utilisée.

17. Procédé selon l'une quelconque des revendications précédentes, **caractérisé en ce que** le catalyseur est agencé dans le réacteur sous la forme d'un lit fixe.

18. Procédé selon l'une quelconque des revendications précédentes, **caractérisé en ce qu'**il est réalisé en continu.

19. Procédé selon les deux revendications précédentes, **caractérisé en ce que** la réaction est tout d'abord réalisée à une température dans la plage allant de 80 à 160 °C, puis à une température dans la plage allant de 180 à 220 °C.

20. Procédé selon l'une quelconque des trois revendications précédentes, **caractérisé en ce que** la réaction a lieu dans un réacteur tubulaire.

21. Procédé selon l'une quelconque des quatre revendications précédentes, **caractérisé en ce que** la réaction a lieu en un mode de circulation de gaz.

22. Procédé selon l'une quelconque des revendications précédentes, **caractérisé en ce que** la DEOA est utilisée sous la forme d'une solution aqueuse.

23. Procédé selon l'une quelconque des revendications précédentes, **caractérisé en ce que** l'amine primaire III est utilisée sous la forme d'une solution aqueuse.

24. Procédé selon l'une quelconque des revendications précédentes, **caractérisé en ce que** la réaction est réalisée à une charge du catalyseur dans la plage allant de 0,3 à 0,7 kg DEOA/ (l_{cat}·h).

25. Procédé selon l'une quelconque des revendications précédentes, **caractérisé en ce que** la réaction est réalisée à une charge du catalyseur dans la plage allant de 100 à 1 000 litres normés d'hydrogène/ (l_{cat}·h).

26. Procédé selon l'une quelconque des revendications précédentes, pour la fabrication d'une mono-N-alkyl-pipérazine de formule I, dans laquelle R¹ signifie méthyle, éthyle ou 2-(2-hydroxy-éthoxy)-éthyle, par mise en réaction de diéthanolamine (DEOA) de formule II avec une amine primaire de formule H₂N-R¹ (III).

27. Procédé selon l'une quelconque des revendications précédentes, **caractérisé en ce qu'**à partir du produit de la réaction, par distillation
(i) tout d'abord l'amine primaire III éventuellement non réagie est séparée par la tête,
(ii) l'eau est séparée par la tête,
(iii) les produits secondaires éventuellement présents ayant un point d'ébullition inférieur à celui du produit du procédé I sont séparés par la tête,
(iv) le produit du procédé mono-N-alkyl-pipérazine I est séparé par la tête, les produits secondaires éventuellement présents ayant un point d'ébullition supérieur à celui du produit du procédé I et la DEOA (II) non réagie éventuellement présente restant dans le fond.

28. Procédé selon la revendication précédente, **caractérisé en ce que**, par distillation,
(v) la DEOA (II) non réagie éventuellement présente et/ou l'alkylaminoéthyléthanolamine éventuellement présente en tant que produit secondaire de formule IV 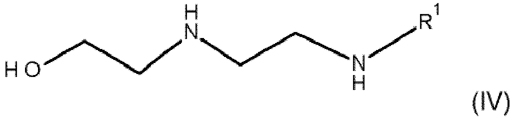 sont séparés par la tête à partir du fond de l'étape iv, et recyclés dans la réaction.

29. Procédé selon l'une quelconque des deux revendications précédentes, **caractérisé en ce que** l'amine primaire III séparée à l'étape i d'une pureté de 90 à 99,9 % en poids est recyclée dans la réaction, une partie de l'amine III étant de préférence évacuée.

30. Procédé selon l'une quelconque des revendications 1 à 26, pour la fabrication de mono-N-alkyl-pipérazine de formule I avec R¹ = 2-(2-hydroxy-éthoxy)-éthyle, **caractérisé en ce qu'**à partir du produit de la réaction, par distillation
(i) tout d'abord, l'eau est séparée par la tête,
(ii) l'amine primaire III (= ADG) éventuellement non réagie est séparée par la tête,
(iii) les produits secondaires éventuellement présents ayant un point d'ébullition inférieur à celui du produit de procédé I sont séparés par la tête,
(iv) le produit de procédé mono-N-alkyl-pipérazine I est séparé par la tête, les produits secondaires éventuellement présents ayant un point d'ébullition supérieur à celui du produit de procédé I et la DEOA (II) non réagie éventuellement présente restant dans le fond.

31. Procédé selon la revendication précédente, **caractérisé en ce que**, par distillation,
(v) la DEOA (II) non réagie éventuellement présente et/ou l'alkylaminoéthyléthanolamine éventuellement présente en tant que produit secondaire de formule IV 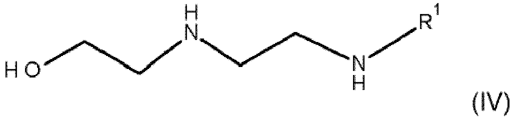 sont séparés par la tête à partir du fond de l'étape iv, et recyclés dans la réaction.

32. Procédé selon l'une quelconque des deux revendications précédentes, **caractérisé en ce que** l'ADG séparé à l'étape ii d'une pureté de 90 à 99,9 % en poids est recyclé dans la réaction, une partie de l'ADG étant de préférence évacuée.
